# EUROPEAN PATENT SPECIFICATION

(11) **EP 3 763 282 B1**
(45) Date of publication and mention of the grant of the patent: **29.10.2025**
(21) Application number: 19764141.8
(22) Date of filing: 28.02.2019
(51) Int. Cl.: A61B 3/10, A61B 3/12

(54) **OCULAR FUNDUS IMAGING DEVICE**
VORRICHTUNG ZUR BILDGEBUNG DES AUGENHINTERGRUNDES
DISPOSITIF D'IMAGERIE DU FOND DE L'OEIL

(30) Priority: 06.03.2018 JP 2018039471
(43) Date of publication of application: 13.01.2021
(73) Proprietor: Nidek Co., Ltd., Gamagori-shi, Aichi, 443-0038 (JP)
(72) Inventor: MURASE, Yuji, Gamagori-shi Aichi 443-0038 (JP); FUJIU, Kenshiro, Gamagori-shi Aichi 443-0038 (JP); MIZUNO, Katsuyasu, Gamagori-shi Aichi 443-0038 (JP); ITO, Shohei, Gamagori-shi Aichi 443-0038 (JP); AKITA, Junichi, Gamagori-shi Aichi 443-0038 (JP)
(74) Representative: Hoefer & Partner Patentanwälte mbB
(86) International application number: PCT/JP2019/007760
(87) International publication number: WO 2019/172062

(56) References cited:
- JP-A- 2015 091 309
- JP-A- 2017 189 219
- JP-A- 2017 196 210
- JP-A- 2017 225 599
- US-A1- 2017 332 898

## Description

### Technical Field

The present disclosure relates to a fundus imaging device.

### Background Art

In the related art, a fundus imaging device that captures a fundus image of a subject eye is known. For example, in Patent Literature 1, a device in which a region on a fundus to be imaged is switched between a first angle of view and a second angle of view that is larger, is disclosed. In Patent Literature 1, a lens system is inserted and removed between a main body of the device and the subject eye, and an objective optical system is therefore changed and then an imaging range is increased or decreased.

US 2017 / 0 332 898 A1 describes an optical tomographic imaging apparatus with an optical system which includes a focus lens configured to focus a measuring light on the object to be inspected. The optical tomographic imaging apparatus includes a unit configured to compensate, when an optical member for changing a field angle is inserted between a scanning unit and the object to be inspected in order to change the field angle of an acquiring area of a tomographic image, a change in a focus position of the focus lens in association with the inserting.

JP 2015 091309 A describes an ophthalmological photographing device in which a control part acquires a first eyeground image and a second eyeground image (partial image) having resolution higher than that of the first eyeground image photographed about a portion of the first eyeground image. In addition, the control part combines the second eyeground image with an image area (range of interest) on the first eyeground image corresponding to the second eyeground image, and displays a composite image of the first eyeground image and the second eyeground image on a monitor.

JP 2017 189219 describes an ophthalmologic imaging apparatus which includes a data collecting part, an image forming part, a field angle changing part, a beam size changing part, and a control part. The data collecting part scans the ocular fundus of an eye to be examined using a light beam and collects data. The image forming part forms an image of the ocular fundus based on the data collected by the data collecting part. The field angle changing part has a configuration for changing the field angles. The beam size changing part changes the size of the light beam. The control part controls the field angle changing part and the beam size changing part in a linked manner.

### Citation List

### Patent Literature

Patent Literature 1: JP-A-2016-123467

### Summary of Invention

However, by switching of the angle of view, optical feature amounts other than the angle of view also change. Therefore, if imaging is performed without changing a control amount of the optical system before and after switching of the angle of view, it may not be possible to obtain a good image of the fundus at each angle of view.

In addition, there are various methods for capturing the image of the fundus, an image captured with an angle of view different from that in the related art, and which is obtained using a couple of imaging methods, is found to be not so useful and it is considered that the priority of imaging is clinically low. In addition, there may be an imaging method in which it is difficult to obtain a clinically meaningful image due to hardware restrictions associated with the switching of the angle of view.

An object of the present disclosure is to solve at least one of the problems in the related art, and to satisfactorily capture the image of the fundus both before and after switching of the angle of view.

This object is solved by the features of the independent claim. The dependent claims contain advantageous embodiments of the present invention.

According to the present disclosure, the image of the fundus can be satisfactorily captured both before and after switching of the angle of view.

### Brief Description of Drawings

[FIG. 1] Fig. 1 is a diagram illustrating an OCT optical system in an application example.
[FIG. 2] Fig. 2 is a diagram illustrating a retracted state of an attachment optical system, which is an SLO optical system in the application example.
[FIG. 3] Fig. 3 is a diagram illustrating a attached state of the attachment optical system, which is the SLO optical system in the application example.
[FIG. 4] Fig. 4 is a block diagram illustrating an electrical configuration of a fundus imaging device.
[FIG. 5] Fig. 5 is a diagram illustrating a relationship between a driving range in a diopter correction unit and a diopter correction amount in each mode.
[FIG. 6] Fig. 6 is a diagram for explaining a control for changing a focusing target position in a front imaging optical system.
[FIG. 7] Fig. 7 is a diagram for explaining the control for changing a focus target position in the OCT optical system.
[FIG. 8] Fig. 8 is a diagram illustrating a selection screen of an imaging method.

### Description of Embodiments

### <Summary>

Hereinafter, embodiments of the present disclosure will be described with reference to the drawings. Unless otherwise specified, embodiments using a fundus imaging device (an example of an ophthalmologic device) will be described.

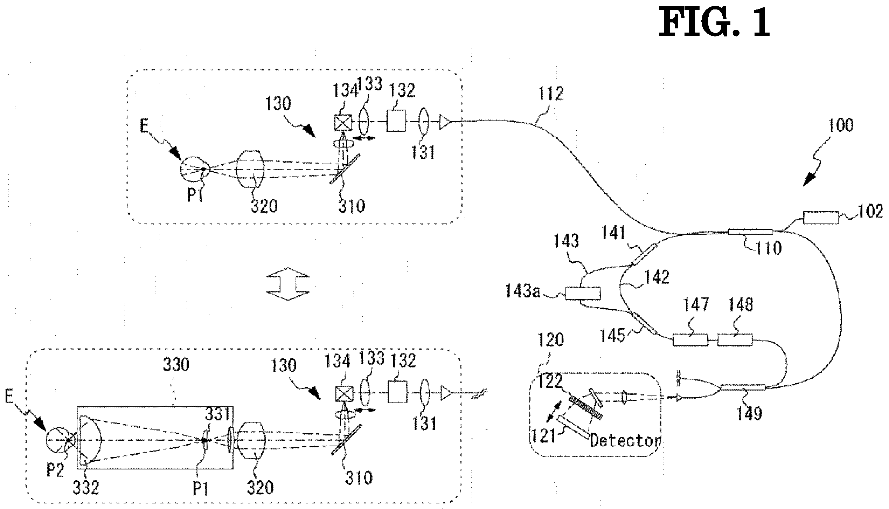

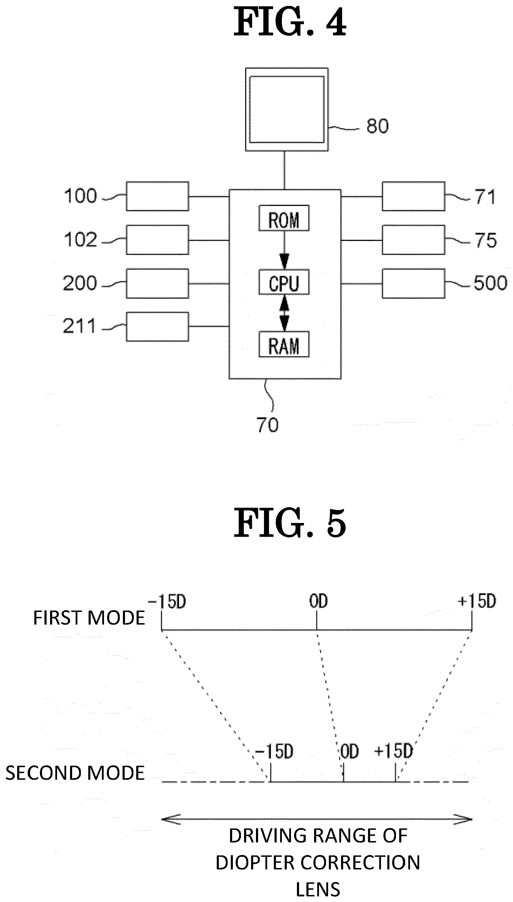

The fundus imaging device includes at least an imaging optical system (see Figs. 1 to 3), an angle-of-view switching unit, and a control unit (see Fig. 4). The control unit may be a processor that manages control of the device.

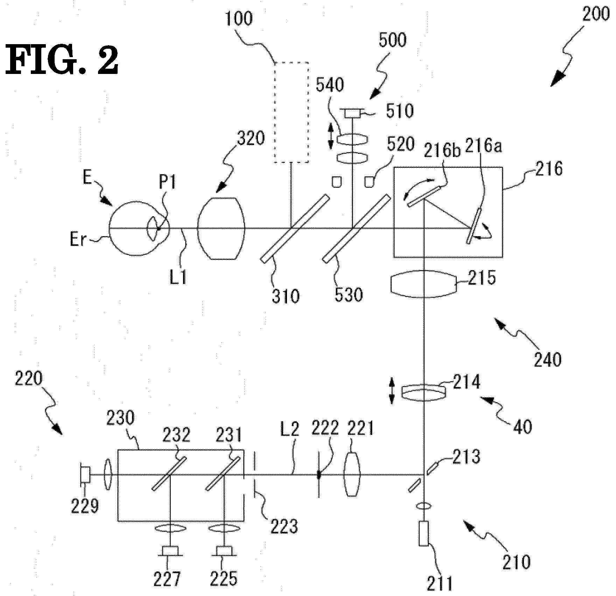

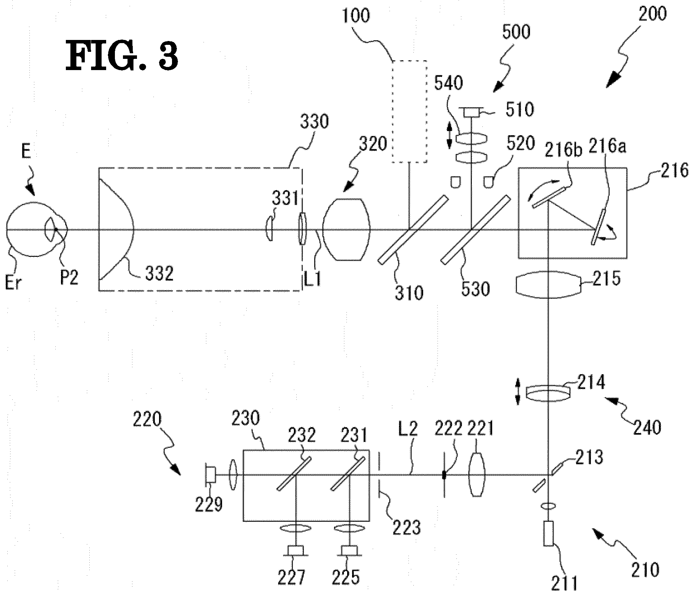

The imaging optical system irradiates a fundus of a subject eye with light through an objective optical system. In addition, the imaging optical system images the fundus of the subject eye based on the return light from the fundus. The imaging optical system includes a light receiving element that receives the return light of the imaging light from the fundus, and acquires the image of the fundus based on the signal from the light receiving element. The imaging optical system may include a front imaging optical system (see Figs. 2 and 3) that captures a front image of the fundus, may include the OCT optical system (refer to Fig. 1) that acquires OCT data of the fundus based on the spectral interference signals between the return light and the reference light, or may include both. In the present embodiment, it can be considered that both the front imaging optical system and the OCT optical system are included in the imaging optical system. In this case, the objective optical system is shared by the front imaging optical system and the OCT optical system.

The OCT optical system acquires the OCT data based on the optical interference between the light applied onto the fundus and the reference light. The OCT optical system may be an FD-OCT. An SD-OCT, an SS-OCT, and the like are known as the FD-OCT. In the description hereafter, the SD-OCT will be used unless otherwise specified. However, not necessarily limited to the above, the OCT optical system of each type may be adopted as a part or all of the imaging optical system.

The objective optical system may be a refraction system, a reflection system, or may a combination of both. In the description hereafter, for the sake of convenience, the objective optical system will be described as having a plurality of lenses.

The angle-of-view switching unit switches the angle of view in the imaging optical system by changing at least the objective optical system. In other words, the angle-of-view switching unit varies the magnification of the imaging optical system.

The angle-of-view switching unit may switch the angle of view by changing the lens configuration in the objective optical system, for example. As an example, the imaging angle of view may be selectively switched to any one of the two imaging angle of views predetermined by attachment and detachment (inserting and removing) of an attachment optical system (refer to Figs. 1 and 3) such as a lens attachment. Here, the narrower imaging angle of view is referred to as a "first angle of view", and the wider imaging angle of view is referred to as a "second angle of view". For example, the first angle of view may be less than 90° and the second angle of view may be equal to or larger than 90°. For example, the first angle of view may be approximately 45° to 60° and the second angle of view may be approximately 90° to 150°.

However, the method of switching the angle of view of the imaging optical system is not limited to the attachment and detachment (inserting and removing) of the attachment optical system. For example, the angle of view may be switched by exchanging a part or all of the objective optical system. In addition, the angle of view may be switched by a zoom mechanism that can change the arrangement of optical elements in the objective optical system.

### <Mode Change in accordance with Switching of Angle of View>

The control unit (refer to Fig. 4) changes an imaging mode between a first mode in which the fundus is imaged at the first angle of view and a second mode in which the fundus is imaged at the second angle of view in accordance with switching of the angle of view by the angle-of-view switching unit. That is, the imaging conditions and the control of the device are changed.

### <Switching of Signal Intensity of Received Light Signal>

The control unit performs various switches relating to an intensity of received light signal between the first mode and the second mode. As the angle of view becomes wider, the area of the fundus onto which the light is applied increases. Therefore, as the angle of view becomes wider, the contrast of the fundus image tends to decrease. For this reason, in the second mode, an amount of light used for fundus imaging or a gain of the received light signal based on the return light may be increased compared to that in the first mode. The amount of light may be increased or decreased based on the control of the light source. In addition, a light limiting member in a light receiving optical path may be controlled to increase the passing ratio of the return light. Examples of the light limiting member include various apertures and filters. For example, by changing a size of the opening of the aperture or by selectively arranging one of a plurality of filters having transmission characteristics different from each other on the optical path, the passing ratio of the return light may be increased in the second mode compared to that in the first mode.

The parameters such as amount of light and gain do not need to be fixed in each mode. The parameters may be manually changeable. For example, initial values (initial settings) of various parameters in each mode may be changed interlocking with the mode as described above. The initial value (initial setting) is not limited to the initial value of the amount of light and the gain, but for example, initial values of various parameters such as an amount of driving of the optical element in the diopter correction unit, a presentation position of a fixation lamp, and a dispersion correction amount in OCT also may be similarly changed.

### <Upper Limit Switching of Amount of Irradiation Light>

The fundus imaging device may include a light detection unit. The light detection unit is used for detecting the amount of light applied from the imaging optical system to the subject eye (imaging light or observation light). The light amount detection unit may include a light receiving element such as a photodiode. The light receiving element may detect the amount of light from the light source of the imaging optical system. For example, the amount of light may be detected by branching and guiding the light from the light source to the light receiving element, or by receiving the stray light in the housing by the light receiving element, or other methods may be used.

At this time, the control unit compares the amount of light detected by the light amount detection unit with a predetermined threshold value. If the detected amount of light is equal to or less than the threshold value, the control unit allows the irradiation of the subject eye with the light. On the other hand, if the detected amount of light is larger than the threshold value, the control unit inhibits the irradiation of the subject eye with the light. As an inhibition method, a shutter unit arranged between the light source and the subject eye may be closed, or the output from the light source may be reduced, or other methods may be used. As a result of blocking, it is possible to prevent an excessive load from being applied to the subject eye.

Here, it is conceivable that a light flux diameter of the light applied to the subject eye varies in between the first mode and the second mode. In this case, it is considered that the light density in the intermediate light-transmissive body of the subject eye varies in between the first mode and the second mode.

Therefore, in the present embodiment, the control unit may change the above-described threshold value between the first mode and the second mode. More specifically, the control unit may reduce the threshold value in the second mode in which the angle of view is wider in comparison with the threshold value in the first mode.

### <Switching of Focus Control>

The control unit changes a control of the diopter correction unit between the first mode and the second mode. The diopter correction unit includes one or a plurality of optical elements (lens and prism, and the like), and performs the diopter correction according to the subject eye by driving the optical elements. The diopter correction unit may include a driving unit (a driver) that drives the optical element. Various optical systems are known for the diopter correction unit, and any of them may be applied. For example, a unit having a plurality of lenses, a unit having a Badal optical system, a unit having a variable focus lens, or the like are known as the diopter correction unit.

The diopter correction is performed, for example, by detecting a focus state (state of diopter correction) from the image of the fundus acquired through the imaging optical system, and driving the optical element based on the detected focus state. The control of the diopter correction may be a feedback control, or may be performed by a repeated drive control.

The focus state may be detected based on contrast information in the image of the fundus, for example. A focus index may be projected onto the fundus, and the focus state may be detected based on the focus index projected onto the fundus. In addition, the focus state may also be detected based on the region of the fundus tissues in the image. In this case, the focus index is not necessarily needed.

### <Switching of Unit Correction Amount in Diopter Correction>

For example, the control unit sets the amount of driving of the optical element per step in each of the first mode and the second mode. In this way, the diopter correction step (unit: D) in the diopter correction unit is increased in the second mode compared to that in the first mode. For example, the diopter correction step is switched so as to be 0.25D step in the first mode and 1.0D step in the second mode (refer to Fig. 5).

In the second mode, the difference in height of the imaging range caused by the curvature of the fundus is larger than that in the first mode. Therefore, the diopter correction step (unit: D) may be increased according to the angle of view. This enables the quick diopter correction even in the second mode in which the difference in height of the imaging range increases.

In addition, the depth of field may be greater in a case of imaging in the second angle of view than in a case of the first angle of view. In this case, by increasing the diopter correction step, it is possible to promptly guide to a good focused state.

The control unit may decrease the amount of driving of the optical element per diopter correction step (unit: D) in the diopter correction unit in the second mode compared to that in the first mode. As the angle of view increases, the diopter (correction amount) changes to be increased with respect to the amount of driving of the optical element. Therefore, if the amount of driving per step is the same between the first mode and the second mode, since the change in the diopter in one step becomes too large with respect to the depth of field in the second mode, in some cases, it becomes difficult to perform the appropriate diopter correction. On the contrary, in the present embodiment, it is easy to appropriately set the correction amount (D) per step in each mode according to the depth of field in each mode.

### <Switch of Detection Region on Image>

First, a case where the state of the diopter correction is detected based on an observation image by the front image of the fundus acquired through the imaging optical system, and the diopter correction is performed based on the result of detection, will be described.

The control unit sets the detection region for all or part of the observation image. Then, the control unit detects the state of the diopter correction based on the detection region (specifically, based on the image information in the detection region).

Here, in the present embodiment, for example, the control unit may make the ranges occupied by the detection regions on the observation image in the first mode and in the second mode different from each other. That is, the positions (coordinates) and sizes of the detection regions on the observation image may be different from each other.

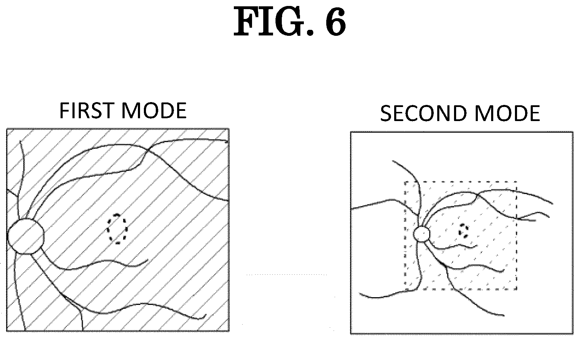

As a specific example, in the case of first mode, the entire image of the fundus image obtained in the first mode may be used as the detection region. In addition, in the case of the second mode, the center part of the image may be the detection region (refer to Fig. 6). The detection region in each mode may match each other on the fundus. For example, in the specific example described above, the center part of the image, which is the detection region in the second mode, may be a region corresponding to the angle of view in the first mode. Because the detection regions match each other on the fundus, the image obtained in the first mode and the image obtained in the second mode have a similar appearance in the detection region, which is useful for the comparison of the two.

The detection region in the second mode is not necessarily limited to the image central part. The detection region in the second mode may include at least the image peripheral part. In this case, a region (second region) outside the region (first region) corresponding to the first mode observation image may be set as the detection region. In addition, in this case, the detection region in the second mode may be the entire image. Furthermore, in this case, the detection region in the first mode may be the image central part. Specific examples of the image central part include the macula or a peripheral region of an optic disk.

### <Switching of Target Part of Focusing on Fundus>

In addition, the control unit is configured to drive the diopter correction unit to guide the different parts of the fundus between the first mode and the second mode to be focused as the target. That is, the so-called JUST PIN (in-perfect-focused) part may be switched between the first mode and the second mode. The part of the fundus here may be a part that can be grasped in the front image or a part that can be grasped in a tomographic image based on the OCT data (for example, a layer of fundus).

In second mode, the difference in height due to the curvature of the fundus is larger. Therefore, in the second mode, if the same part as in the first mode is guided to be focused as the focusing target, it is conceivable that the entire image may not be included in the depth of field. Therefore, the different parts of the fundus between the first mode and the second mode may be guided to be focused as a target. In this way, in each of the first mode and the second mode, the fundus image can be captured in a desired focused state.

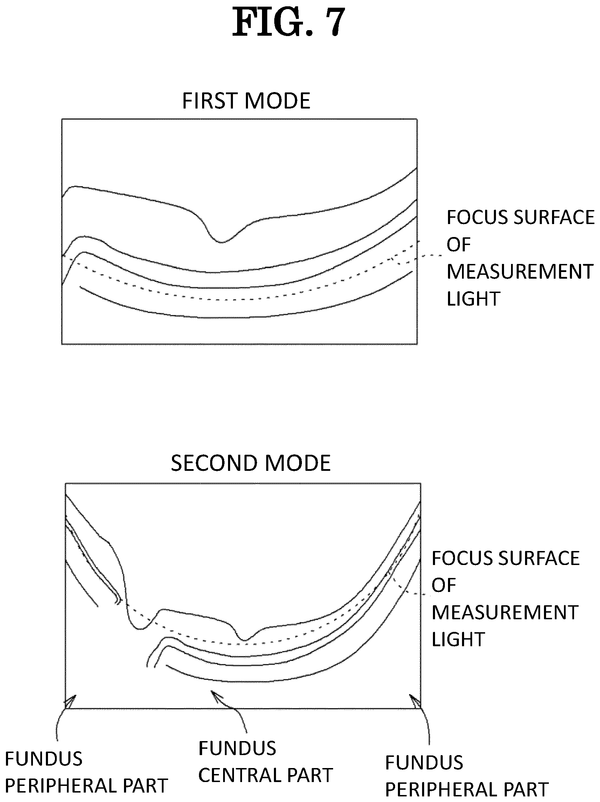

When acquiring the OCT data through the imaging optical system, the control unit may guide different layers of the fundus between the first mode and the second mode to be focused as a target part (see Fig. 7). At this time, the control unit may set the target part in the second mode to a shallower layer side than the target part in the first mode. This makes it easier for a wider range of fundus to be included in the depth of field in each of the first mode and the second mode.

In addition, the control unit is configured to drive a diopter correction unit to guide the same parts of the fundus between the first mode and the second mode to be focused as the target. When targeting the same part of the fundus, the processing for specifying the target part (in other words, the detection processing) may be different from each other between the first mode and the second mode.

### <Switching between Static Correction Control and Dynamic Correction Control>

When acquiring the OCT data, in first mode, the diopter correction may be performed with a static correction amount (constant correction amount) regardless of the scanning position of the optical scanner, and in the second mode, the diopter correction may be performed with a dynamic correction amount according to the scanning position of the optical scanner on the fundus. In the dynamic correction, the diopter correction amount is changed between at least at the fundus central part and at the fundus peripheral part. As a result, even in the second mode, it becomes easy to obtain good focus in the entire imaging range.

### <Interlocked Control of Fixation Lamp when Focusing on Fundus Peripheral Part>

In addition, as a result of guiding the focus point to the fundus peripheral part in the second mode, it is conceivable that the fixation lamp seen by the examiner may be blurred. Therefore, for example, if the diopter correction of the fixation optical system can be performed independently of the imaging optical system, the diopter correction amount in the fixation optical system can be deviated from the diopter correction amount in the imaging optical system according to the region to be focused on the fundus.

### <Change of Reference Amount of Diopter Correction>

The control means may control the diopter correction unit, and may set reference amounts of correction that are different from each other between the first mode and the second mode. The final correction amount may be set from the ranges before and after the reference correction amount. More specifically, the focused state may be detected in the range before and after a predetermined reference correction amount, being interlocking with the mode switch, after the position of the optical element for diopter correction is changed or at the time of changing. The final correction amount may be set based on the result of detection at this time.

### <Switching relating to Fixation Lamp>

The control unit may change the lighting control of a fixation target between the first mode and the second mode. For example, the control unit may switch the selectable fixation position between the first mode and the second mode. The fixation position is selected, for example, based on the scanning of the operation unit by the examiner. Among the plurality of presentation positions selectable in the first mode, the presentation positions selectable in the second mode may be limited. For example, if two fixation positions having different distances from the central fixation position are selectable in the first mode, in the second mode, it is considered that there is little meaning in selecting one position closer to the central fixation position. Therefore, in the second mode, regarding the above two fixation positions, only the other fixation position farther from the central fixation position may be selectable.

The fixation target is projected onto the subject eye by the fixation optical system included in the fundus imaging device. Various aspects of the fixation optical system are known, and at least a part of the imaging optical system may be independent. In addition, if the imaging optical system is an SLO optical system that performs a two-dimensional laser scanning, by temporarily turning on visible light at the timing when a predetermined presentation position is scanned with the laser, the SLO optical system may also serve as the fixation optical system.

### <Switching of Selectable Imaging Method>

The control unit may make the imaging methods selectable in the first mode and the second mode different from each other. For example, each imaging method is different from each other in at least one of an optical system used for imaging, the type according to the presence or absence of a contrast agent, the signal processing (image processing method), and the like. For example, the imaging method may be classified into front image imaging and the OCT imaging, or in addition, the front image imaging may be classified into reflection imaging and fluorescence imaging, and the OCT imaging may be classified into normal OCT imaging and functional OCT imaging.

Due to various restrictions (for example, restrictions on hardware, restrictions on optical safety, and the like), it is difficult to match the imaging conditions between the first mode and the second mode. In addition, in some of the imaging methods, it may be difficult to obtain a clinically meaningful image in one mode. In addition, in some of the imaging methods, a clinical priority may be low. Therefore, the control unit makes the imaging methods selectable between the first mode and the second mode be different from each other, and then, suppresses the inappropriate imaging in each mode.

In each of the first mode and the second mode, the control unit selects an imaging method based on, for example, an operation of the operation unit (input interface) by the examiner. When receiving the operation, the control unit may display a widget (controller) corresponding to the imaging method on the monitor in advance. As the widget, various items such as buttons, icons, and lists can be appropriately selected.

The control unit may switch and display the widget for selecting the imaging method interlocking with the switching between the first mode and the second mode. For example, the widget corresponding to the imaging method that can be selected in the imaging mode at that time may be displayed, while the widget corresponding to the non-selectable imaging method may not be displayed. In addition, for example, the widget corresponding to the selectable imaging method and the widget corresponding to the non-selectable imaging method may be simultaneously displayed on the monitor as the different display modes.

### <Control of Limiting OCT Imaging>

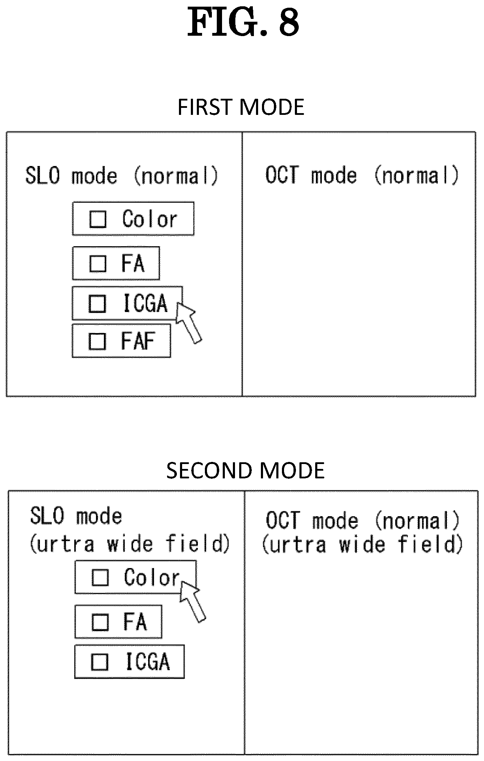

If the imaging optical system includes the front imaging optical system and the OCT optical system, the control unit may be able to select both the front image capturing and the OCT data acquisition in one of the first mode and the second imaging mode, and in the other mode, may be able to select the front image capturing, and further, not to select the OCT data acquisition (for example, refer to Fig. 8).

In the present embodiment, since the angle of view can be switched to the second angle of view by adding the attachment optical system to the optical system of the first angle of view, at the time before and after switching of the angle of view, the length of optical path from the light source to the subject eye largely changes, and thus, it is necessary to compensate the change. However, in the related art, an OPL adjustment unit formed in the reference optical system of the OCT optical system or in a measurement optical system compensates (corrects) an error (individual difference) in an eye axial length, and an adjustment range was not assumed, so as can compensate the change of length of optical path due to the attachment and detachment (insertion and removal) of the attachment optical system. Therefore, it is conceivable that, in one imaging mode, the OCT data can be appropriately acquired, but in the other mode, the OCT data cannot be appropriately acquired. Therefore, in one mode that is outside the OPL adjustment range, it is possible to prevent the inappropriate imaging from being performed in advance by disabling the selection of the OCT data acquisition.

Of course, as in the application examples described later, as long as the change of the length of optical path due to the attachment and detachment (insertion and removal) of the attachment optical system can be compensated sufficiently, it is not necessary to perform such a control of limiting the OCT imaging.

### <Control of Limiting Spontaneous Fluorescence Imaging in Second Mode>

As described above, as the angle of view becomes wider, since the contrast of the fundus image becomes lower, and further, the spontaneous fluorescence from the fundus is weak compared to fundus reflected light and the fluorescence by the contrast agent, it is considered that it may be difficult to obtain a spontaneous fluorescence image in the second mode. Therefore, in the first mode, the control unit may be able to select both the front image capturing based on the fundus reflected light and the spontaneous fluorescence image capturing based on the fundus spontaneous fluorescence, and in the second mode, may not be able to select the spontaneous fluorescence image capturing, and may be able to select the front image capturing based on fundus reflected light. In this way, it is possible to prevent inappropriate imaging from being performed in advance.

### <Control of Limiting Moving Image Capture in Second Mode>

Currently, in the fundus examination, a case where the visible light is applied onto the fundus and the fundus is imaged by a moving image is limited to the case of the contrast imaging (mainly at the initial stage of contrast). In the contrast imaging, the fundus central part is mainly observed, and in order to observe leakage of the contrast agent from the capillaries, a high-definition image is desired. Therefore, it is hard to consider to select the second angle of view rather than the first angle of view. Furthermore, since the contrast imaging imposes a heavy burden on the examinee, the failure in imaging cannot be allowed.

Therefore, in the first mode, both the front image capturing as still images and the front image capturing as moving images may be selectable, and in the second mode, the front image capturing as the moving image may not be selectable and the front image capturing as the still image may be selectable. The moving image capturing that cannot be selected in the second mode may be allowed at least in a case of the contrast imaging, and in a case of the moving image capturing using other imaging methods.

### <Switching of Presence or Absence of Mask on Front Image>

In addition, between the first mode and the second mode, it may be switched whether to add a mask to the captured front image. In other words, the front image captured in the first mode may be displayed or stored in the memory with adding a mask to the peripheral part of the image and the front image captured in the second mode may be displayed or stored in the memory without adding a mask.

### <Application Example>

Hereinafter, a typical application example of the present disclosure will be described with reference to the drawings. First, an overall configuration of a fundus imaging device 1 will be described with reference to Figs. 1 to 3. In the present application example, the fundus imaging device 1 includes an OCT optical system 100 (see Fig. 1) that acquires the OCT data of the fundus, and a front imaging optical system 200 (see Figs. 2 and 3) that captures the front image of the fundus. The two optical systems share an objective optical system. In the present application example, the OCT optical system 100 has, for example, a spectral domain type OCT (SD-OCT) as a basic configuration. Furthermore, in the present application example, the front imaging optical system is a scanning laser ophthalmoscope (SLO) optical system. In the present application example, the front imaging optical system 200 also serves as the fixation optical system.

The fundus imaging device 1 also includes a calculation controller (calculation control unit) 70. In addition, the fundus imaging device 1 may be provided with a memory 72, a monitor 75, an operation unit 80, and the like. In addition, the calculation controller (hereinafter, the control unit) 70 is connected to an OCT light source 102, the OCT optical system 100, a laser light source 201, the front imaging optical system 200, and the like. An operation unit 80 may be a touch panel, a mouse, a keyboard, or the like. An operation unit 75 may be a separate device from the fundus imaging device 1. The control unit 70 may control each unit based on an operation signal output from the operation unit 80. To the operation unit 80, for example, an operation for selecting an imaging mode, an operation for releasing, or the like may be input.

### < OCT Optical System>

The OCT optical system 100 guides measurement light to an eye E by a light guide optical system 130. The OCT optical system 100 guides the reference light to the reference optical system 140. The OCT optical system 100 causes a detector (light receiving element) 120 to receive an interference signal light acquired by the interference between the measurement light reflected by the eye E and the reference light. The OCT optical system 100 is mounted in a housing (a main body of the device) (not illustrated), and may perform the alignment of the subject eye by three-dimensionally moving the housing with respect to the eye E using a well-known alignment moving mechanism through an operation member such as a joystick.

An SD-OCT method is used for the OCT optical system 100. A light source that emits a light flux having low coherence length is used as the OCT light source 102, and a spectral detector that spectrally detects a spectral interference signal for each wavelength component is used as the detector 120.

A coupler (splitter) 104 is used as a first light splitter, and splits the light emitted from the OCT light source 102 into a measurement optical path and a reference optical path. The coupler 110 guides the light from the OCT light source 102 to an optical fiber 112 at the measurement optical path side, and to the reference optical system 140 at the reference optical path side.

### <Light Guide Optical System>

The light guide optical system 130 is provided to guide the measurement light to the eye E. The light guide optical system 130 may be sequentially provided with, for example, the optical fiber 112, a collimator lens 131, a variable beam expander 132, an optical scanner 134, and an objective lens 320. In this case, the measurement light is emitted from the emission end of the optical fiber 112 and becomes a parallel beam by the collimator lens 131. After that, the measurement light that became the parallel beam goes toward the optical scanner 134 through the focusing lens 133 in a state of having a desired light flux diameter by the variable beam expander 132. The light that has passed through the optical scanner 134 is applied onto the eye E through the objective lens 320. A first turning point P1 is formed at a position conjugate with the optical scanner 134 with respect to the objective lens 320. Since the anterior segment is positioned at the turning point P1, the measurement light reaches the fundus without vignetting. In addition, the fundus is scanned with the measurement light according to the operation of the optical scanner 134. At this time, the measurement light is scattered and reflected by the tissue of the fundus.

The eye E may be scanned with the measurement light in the XY direction (transverse directions) by the optical scanner 134. The optical scanner 134 is, for example, two Galvano-mirrors, and the reflection angle thereof is arbitrarily adjusted by the drive mechanism. The reflection (traveling) direction of the light flux emitted from the OCT light source 102 changes, and the scanning is performed in an arbitrary direction on the fundus. As the optical scanner 134, for example, a reflection mirror (a galvano-mirror, a polygon mirror, a resonant scanner), or an acoustic optical element (AOM) that changes the traveling (deflection) direction of the light, may be used.

The scattered light (reflected light) of the measurement light from the eye E retraces the path at the time of the projection, and enters the optical fiber 112 to reach the coupler 110. The coupler 110 guides the light from the optical fiber 112 to the optical path toward the detector 120.

### <Attachment Optical System>

In the OCT device in the application example, the attachment optical system 330 is inserted and removed between the objective lens 320 and the subject eye E in the light guide optical system 130. By the lens attachment including the attachment optical system 330 being attached and detached (insertion and removal) to a housing surface (not illustrated), the attachment optical system 330 is inserted and removed between the objective lens 320 and the subject eye E at the main body side of the device.

The attachment optical system 330 may include a plurality of lenses 331 and 332. At least the lens 164 bends the measurement light that passed through the first turning point P1 toward the optical axis L, and then, a second turning point P2 is formed at a position conjugate with the optical scanner 134 with respect to the attachment optical system 330 and the objective optical system 158. That is, the attachment optical system 330 is an optical system that relays the turning point P1 to the turning point P2.

In the present application example, the turning amount of the measurement light at the second turning point P2 is larger than the turning amount at the first turning point P1. For example, if the turning amount is indicated by a solid angle, the solid angle at the second turning point P2 is increased to be equal to or more than twice the solid angle at the first turning point P1. In the present application example, in a retracted state (the first mode), the scanning can be performed within a range of approximately 60°, and in an insertion state (the second mode), the scanning can be performed within a range of approximately 100°.

The variable beam expander 132 is a light flux diameter adjustment unit in the application example. As an example, the variable beam expander 132 may be configured to include a plurality of lenses forming a bilateral telecentric optical system, and the light flux diameter may be switched by changing the lens interval by an actuator. The variable beam expander 132 adjusts the light flux diameter of the measurement light based on the instruction from the control unit 70.

For example, if it is assumed that the light flux diameter of the measurement light guided from the variable beam expander 132 to the optical scanner 134 is constant between the retraction state (the first mode) and the insertion state (the second mode), since a spot size of the measurement light on the fundus is proportional to the angle of view, the resolution in the insertion state is lower than that in the retraction state. Therefore, in the present application example, the control unit 70 drives the variable beam expander 132 according to the insertion and removal of the attachment optical system, and reduces the light flux diameter in the insertion state with respect to the retraction state. The ratio of the light flux diameter in the insertion state and the retraction state (light flux diameter in the variable beam expander 132) is the inverse ratio of the angle of view in the insertion state and the retraction state, and therefore it is possible to suppress the change in the resolution based on the insertion and removal of the attachment optical system 330.

### <Reference Optical System>

The reference optical system 140 generates the reference light that is combined with the fundus reflected light of measurement light. The reference light that passed through the reference optical system 140 is multiplexed with the light from the measurement optical path by the coupler 149, and then, interferes. The reference optical system 140 may be a Michelson type or a Mach-Zehnder type.

The reference optical system 140 illustrated in Fig. 1 is formed of a transmission optical system. In this case, the reference optical system 140 guides the light from the coupler 110 to the detector 120 by transmitting the light without returning. Not limited thereto, the reference optical system 140 may be formed by, for example, a reflective optical system, and may guide the light from the coupler 110 to the detector 120 by reflecting the light from the reflective optical system.

In the present application example, the reference optical system 140 may be provided with a plurality of reference optical paths. For example, in Fig. 1, the reference optical path is branched into an optical path passing through the fiber 142 (first branch optical path in the present application example) by the coupler 141, and an optical path passing through the fiber 143 (second branch optical path in the present application example). The fiber 142 and the fiber 143 are connected to the coupler 145, which couples the two branch optical paths, and the light is incident on the coupler 149 through the reference optical path adjustment unit 147 and the polarization adjustment unit 148.

In the present application example, the reference light from the coupler 110 is simultaneously guided to the fiber 142 and the fiber 143 by the coupler 141. The light passing through either the fiber 142 or the fiber 143 is multiplexed with the measurement light (fundus reflected light) in the coupler 149.

A difference in length of optical path between the fibers 142 and 143, that is, a difference in length of optical path between the first branch optical path and the second branch optical path may be a fixed value. In the present application example, a difference in length of optical path is substantially the same as the difference in length of optical path of the attachment optical system 330.

A reference optical path adjustment unit 147 for adjusting the difference in length of optical path between the measurement light and the reference light may be provided on at least one of the measurement optical path and the reference optical path. An adjustment range of the length of optical path by the reference optical path adjustment unit 147 is preferably set sufficiently short with respect to the difference in length of optical path (in other words, the difference in length of optical path between a first branch optical path and a second branch optical path) between the fiber 142 and the fiber 143.

### <Light Detector>

The detector 120 is provided for detecting interference due to the light from the measurement optical path and the light from the reference optical path. In the present application example, the detector 120 is a spectral detector, and includes, for example, a spectroscope and a line sensor, the measurement light and the reference light multiplexed by the coupler 149 are spectrally separated by the spectroscope, and is received in the different regions (pixels) in the line sensor for each wavelength. As a result, the output for each pixel is acquired as the spectral interference signal.

The curvature of the fundus and the image plane of the measurement light do not always match each other, and in the insertion state of the attachment optical system 150, since a separation between at least one of the fundus central part or the fundus peripheral part increases, it is preferable that a sufficient depth range is secured for the light detector considering the separation.

### <Acquisition of Depth Information>

The control unit 70 processes the spectral interference signal detected by the detector 120 (Fourier analysis) to obtain the OCT data of the subject eye.

The spectral interference signal (spectral data) may be rewritten as a function of wavelength λ, and then, may be converted into a function I (k) having an equal interval with respect to the wave number k (=2π/λ). Alternatively, from the beginning, the function may be acquired as a function I (k) having an equal interval with respect to the wave number k (K-CLOCK technology). The calculation controller may obtain the OCT data in the depth (Z) region by performing Fourier transform on the spectral interference signal in the wave number k space.

Furthermore, the information after the Fourier transform may be represented as a signal including a real number component and an imaginary number component in the Z space. The control unit 70 may obtain the OCT data by obtaining an absolute value of the real number component and the imaginary number component in the signal in the Z space.

Here, the reference light that passed through the first branch optical path and the reference light that passed through the second branch optical path are simultaneously guided to the coupler 149, and each is multiplexed with the measurement light. Since there is a large difference in length of optical path between the first branch optical path and the second branch optical path, which can be said to be a degree same as the length of optical path of the attachment optical system 330, among the reference light that passed through the first branch optical path and the reference light that passed through the second branch optical path, one is likely to interfere with the measurement light, but the other one is less likely to interfere. The spectral interference signal from the detector 120 includes a component due to the reference light that passed through the first branch optical path and a component due to the reference light that passed through the second branch optical path, and among the two types of components, one corresponding to the state of the light guide optical system 130 is obtained as a significantly stronger signal than the other. As a result, it is possible to obtain the good OCT data regardless of the state of the light guide optical system 130. That is, by providing a plurality of reference optical paths having the difference in length of optical path corresponding to the attachment optical system 330, in the OCT device in the application example, the amount of change in the difference in length of optical path between the measurement optical path and the reference optical path, which is caused by the insertion and removal of the attachment optical system 330, is compensated regardless of the state of the light guide optical system 130.

The reference optical path adjustment unit 147 may be controlled, and then, the difference in length of optical path between the measurement optical path and the reference optical path, which is related to the eye axial length of the subject eye E, may be further adjusted.

In the insertion state, since the fundus reflected light of the measurement light from the fundus peripheral part becomes weaker than the reflected light from the fundus central part, the difference in length of optical path between the measurement optical path and the reference optical path may be adjusted by the reference optical path adjustment unit 147 so that a zero delay position of the measurement optical path and the reference optical path overlaps with the desired fundus tissue (for example, retina, choroid, sclera, or the like) in the fundus peripheral part.

In addition, in the example in Fig. 1, the fiber 143 is connected to an attenuator 143a (attenuator). The attenuator 143a is arranged to adjust the amount of light balance between the measurement light and the reference light in the insertion state and the retraction state of the attachment optical system 330. As illustrated in Fig. 1, when the attenuator is arranged on the branch optical path in the reference optical system, the attenuation rate of the attenuator may be constant.

In addition, the attenuator may be arranged at a position other than the branch optical path in the reference optical system. In this case, the attenuation rate in the attenuator may be variable, and the attenuation rate may be changed by the control unit 70 between the insertion state and the retraction state of the attachment optical system 330.

In addition, in the detector 120, the interval between a grading element 121 (for example, diffraction grating, grading lens, or the like) and the line sensor 122 may be changeable, and by changing the interval, the irradiation range of the light for all the pixels of the line sensor (here, the light obtained by combining the measurement light and the reference light) may be increased or decreased. In this way, the resolution in the depth direction can be changed. For example, the irradiation range in the line sensor 122 may be increased in the insertion state with respect to the retraction state. As a result, it is possible to satisfactorily acquire the OCT data at each position of the fundus even in the insertion state.

In addition, when A-scan is performed at an equal angle interval with a pupil as a center, it is conceivable that the density of scan points on the fundus is higher around the fundus than that of the fundus central center part. However, in the retraction state, since there is no big difference in the distance from the turning point to each scan point, each scan point is at substantially equal interval, however, it is conceivable that in the insertion state there will be some sparse and dense that cannot be ignored. Therefore, in the insertion state, the angle interval centered on the turning point, which is the A-scan angle interval for the fundus central part, may be densely arranged with respect to the A-scan angle interval with respect to the fundus peripheral part. In this way, in the insertion state, the position where the OCT data of the fundus is acquired can be set more equally.

In the present application example, the difference in the dispersion amount of the optical system between the measurement optical path and the reference light is corrected by signal processing. Specifically, the correction is performed by applying the correction value stored in the memory in advance in the above-described the spectral interference signal processing. In the present application example, a first correction value corresponding to the retraction state and a second correction value different from the first correction value and corresponding to the insertion state are stored in the memory 71 in advance, the correction value to be applied can be changed according to the state of the light guide optical system. As a result, in the OCT device in the application example, the amount of change of the dispersion amount between the measurement optical path and the reference optical path, which is accompanied by the insertion and removal of the attachment optical system 330, is compensated in each state of the light guide optical system 130. However, the dispersion amount does not necessarily need to be corrected by signal processing, the dispersion amount may be realized by inserting and removing an optical element for dispersion correction with respect to the light projecting and receiving optical path of the measurement light.

The polarization adjustment unit 148 is for adjusting the polarization state (here, the polarization state of the reference light). The state of polarization may also be changed according to the state of attachment and detachment (insertion and removal) of the attachment optical system 330. For example, before and after the attachment and detachment (insertion and removal) of attachment optical system 330, the polarization adjustment unit 148 may be driven as much as a predetermined angle and may change the polarization state.

The control unit may correct the change in the diopter of the optical system due to the attachment and detachment (insertion and removal) of the attachment optical system 330 by controlling a driving unit (a driver) (not illustrated) and displacing the focusing lens 133 according to the angle of view. For example, in the present embodiment, the displacement amount (that is, diopter correction amount) of the focusing lens 133 may be set based on the observation image acquired through the SLO optical system 200. In this case, the diopter correction amount may be controlled interlocking between the OCT optical system 100 and the SLO optical system.

### <SLO Optical System>

Next, the SLO optical system 200 will be described. The SLO optical system 200 acquires a front image of a fundus Er by the fundus Er being scanned with the laser light and receiving the return light of the laser light from the fundus Er.

In the description hereinafter, the fundus imaging device 1 obtains the fundus image by two-dimensionally scanning the laser light focused on the spot on the observation surface based on the operation of the scanning unit (optical scanner). However, the SLO optical system 200 is not limited to the above, and may be a so-called line scan type optical system. In this case, the observation surface is scanned with a line-shaped light flux. In addition, a non-scanning type optical system may be used as the front image imaging optical system instead of the scanning type optical system as in the present application example.

The SLO optical system 200 includes an irradiation optical system 210 and a light receiving optical system 220.

First, an optical system when the imaging angle of view is the first angle of view will be described with reference to Fig. 2. In the present application example, the imaging angle of view is set to the first angle of view when the attachment optical system 330 (see Fig. 3) is not attached.

The irradiation optical system 210 includes a scanning unit 216 and an objective optical system 300. The objective optical system 300 includes at least one lens. In addition, as illustrated in Fig. 2, the irradiation optical system 210 further includes a laser light source 211, a perforated mirror 213, a lens 214 (a part of the diopter correction unit 240 in the present embodiment), and a lens 215.

The laser light source 211 is a light source of the irradiation optical system 210. In the present embodiment, the laser light from the laser light source 211 is used as imaging light. The laser light source 211 in the present embodiment can simultaneously or selectively emit the light of a plurality of colors. As an example, in the present embodiment, the laser light source 211 emits total of four colors of light: three colors in the visible range of blue, green, and red, and one color in the infrared range. The light of each color may be simultaneously emitted in any combination. In the present application example, the light of each color emitted from the laser light source 211 is used for imaging the fundus. In some cases, the fundus image captured based on the fundus reflected light is referred to as a reflection image, and the fundus image captured based on the fluorescence from a fluorescent substance existing in the fundus Er may be referred to as a fluorescence image.

As the reflection image, any one or all of an infrared image, a color image, a red-free image, a monochrome visible image, and the like may be captured. In addition, as the fluorescence image, any one or all of the contrast fluorescence image and the spontaneous fluorescence image may be captured. The contrast fluorescence image may be an image by fluorescence radiation of the contrast agent intravenously injected into the fundus Er, may be, for example, an FA image (fluorescein contrast captured image), or may be an ICGA image (indocyanine green contrast captured image). Furthermore, the spontaneous fluorescence image may be an image by the fluorescence radiation of the fluorescent substance accumulated in the fundus Er, or may be an image by the fluorescence radiation of lipofuscin.

In the present embodiment, the laser light from the laser light source 211 passes through the openings formed in the perforated mirror 213, passes through the lenses 214 and 215, and then, travels toward the scanning unit 216. The laser light reflected by the scanning unit 216 passes through a dichroic mirror 530 and a dichroic mirror 310, passes through the objective optical system 300, and then, and is applied onto the fundus Er of the subject eye E. As a result, the laser light is reflected or scattered at the fundus Er or excites the fluorescent substance existing in the fundus Er to cause the fluorescence to be generated from the fundus. The light described above (that is, reflected and scattered light, the fluorescence, and the like) are emitted from the pupil as the light (return light) from the subject eye accompanying the irradiation of the laser light.

The scanning unit 216 (also referred to as an "optical scanner") is a unit for scanning the laser light emitted from the laser light source 211 on the fundus Er. In the description hereinafter, unless otherwise specified, the scanning unit 216 includes two optical scanners having different scanning directions of the laser light. That is, the scanning unit 216 includes an optical scanner 216a for main scanning (for example, scanning in the X direction) and an optical scanner 216b for sub -scanning (for scanning in the Y direction). In the description hereinafter, the optical scanner 216a for main scanning is a resonant scanner, and the sub-scanning optical scanner 2216b is a galvanometer mirror. However, another optical scanner may be applied to each of the optical scanners 216a and 216b. For example, in addition to other reflection mirrors (the Galvano-mirrors, polygon mirrors, the resonant scanners, MEMS, and the like), an acoustic optical element (AOM) that changes the travel (deflection) direction of light may be applied to each of the optical scanners 216a and 216b.

The objective optical system 300 is an objective optical system of the fundus imaging device 1. The objective optical system 300 is used to irradiate the subject eye E with the laser light scanned by the scanning unit 216, and guide the laser light to the fundus Er. In order to do that, the objective optical system 300 forms a turning point P at which the laser light passing through the scanning unit 216 is turned. The turning point P is formed at a position optically conjugate with the scanning unit 216 with respect to the objective optical system 300, which is a position on a reference optical axis L1 of the irradiation optical system 210. In the present disclosure, "conjugate" is not necessarily limited to a perfect conjugate relationship, and includes "substantially conjugate". In other words, the case where the fundus image is arranged out of position from the perfect conjugate position within the range permitted in relation to the purpose of use (for example, observation, analysis, or the like) of the fundus image is also included in the "conjugate" in the present disclosure. In the present application example, the objective optical system 300 of fundus imaging device 1 is realized only by the lens, but not limited thereto, may be realized by a combination of the lens and the mirror.

The laser light that passed through the scanning unit 216 passes through the objective optical system 300, passes through the turning point P, and is applied onto the fundus Er. Therefore, the laser light that passed through the objective optical system 300 is turned with centered at the turning point P as the scanning unit 216 operates. As a result, in the present embodiment, the fundus Er is two-dimensionally scanned with the laser light. The laser light applied onto the fundus Er is focused at the focus position (for example, the retina surface).

Next, the light receiving optical system 220 will be described. The light receiving optical system 220 includes one or a plurality of light receiving elements. For example, as illustrated in Fig. 2, the light receiving optical system 220 may include a plurality of light receiving elements 225, 227, and 229. In this case, the light from the fundus Er by the laser light applied by the irradiation optical system 210 is received by at least one of the light receiving elements 225, 227, and 229.

As illustrated in Fig. 2, the light receiving optical system 220 in the present embodiment may share each member arranged from the objective optical system 300 to the perforated mirror 213 with the irradiation optical system 210. In this case, the light from the fundus Er retraces the optical path of the irradiation optical system 210 and is guided to the perforated mirror 213. The perforated mirror 213 removes at least a part of a noise light due to the reflection on a cornea of the subject eye E and the optical system inside the device (for example, the lens surface of the objective lens system), and the light from fundus Er is guided to the independent optical path of the light receiving optical system 220.

An optical path branching member that branches the irradiation optical system 210 and the light receiving optical system 220 is not limited to the perforated mirror 213, and other optical members (for example, a half mirror) may be used.

The light receiving optical system 220 in the present embodiment includes a lens 221, a pinhole plate 223, and a light separation unit (a light separation unit) 230 in the reflection optical path of the perforated mirror 213.

The pinhole plate 223 is arranged on the conjugate plane of the fundus and functions as a confocal aperture in the fundus imaging device 1. That is, when the diopter correction unit 240 appropriately corrects the diopter, the light from the fundus Er that passed through the lens 221 is focused at the opening of the pinhole plate 223. The pinhole plate 223 removes the light from the positions other than the focus point (or the focal plane) of the fundus Er, and guides the rest (light from the focus point) to at least one of the light receiving elements 225, 227, and 229.

The light separation unit 230 separates the light from the fundus Er. In the present embodiment, the light from the fundus Er is wavelength-selectively separated by the light separation unit 230. The light separation unit 230 may also serve as an optical branching unit that branches the optical path of the light receiving optical system 220. For example, as illustrated in Fig. 2, the light separation unit 230 may include two dichroic mirrors (dichroic filters) 231 and 232 having different light separation characteristics (wavelength separation characteristics). The optical path of the light receiving optical system 220 is branched into three by the two dichroic mirrors 231 and 232. In addition, one of the light receiving elements 225, 227, and 229 is arranged at the end of each branch optical path.

For example, the light separation unit 230 separates the wavelengths of the light from the fundus Er and causes the three light receiving elements 225, 227, and 229 to receive the light of the wavelength regions different from each other. For example, the light of three colors of blue, green, and red may be received by the light receiving elements 225, 227, and 229 one by one respectively. In this case, a color image may be acquired from the results of light receiving by the light receiving elements 225, 227, and 229.

In addition, the light separation unit 230 may cause the light receiving elements different from each other to receive the fundus spontaneous fluorescence and the infrared light which is the fundus reflected light of the observation light. In this way, the infrared image may be captured at the same time as the fluorescence image. In the present embodiment, the blue light emitted from the laser light source 211 is used as the excitation light for spontaneous fluorescence.

The control unit 70 forms a fundus image based on the received light signals output from the light receiving elements 225, 227, and 229, for example. More specifically, the control unit 70 forms the fundus image in synchronization with the optical scanning by the scanning unit 216. For example, the control unit 70 forms at least one frame (in other words, one sheet) the fundus image for each light receiving element every time the sub-scanning optical scanner 216b reciprocates at n times(n is an integer of 1 or more). Hereinafter, for convenience, unless otherwise specified, it is assumed that a one-frame fundus image based on reciprocation is formed for each round trip of the sub-scanning optical scanner 216b. In the present embodiment, since three light receiving elements 225, 227, and 229 are provided, the control unit 70 generates a maximum of three types of images based on the signals from the respective light receiving elements 225, 227, and 229 every time the sub-scanning optical scanner 216b makes one round trip.

The control unit 70 may display a plurality of frames of fundus images sequentially formed based on the operation of the device as described above on the monitor 75 as an observation image in time series. The observation image is a moving image made of fundus images acquired in substantially real time. Further, the control unit 70 captures (captures) a part of a plurality of fundus images that are sequentially formed as a captured image (capture image). At that time, the captured image is stored in the storage medium. The storage medium in which the captured image is stored may be a non-volatile storage medium (for example, a hard disk, flash memory, or the like). In the present embodiment, for example, the fundus image formed at a predetermined timing (or period) after the output of a trigger signal (for example, a release operation signal) is captured.

Next, the alignment optical system 500 will be described. The alignment optical system 500 in the present embodiment includes, as an example, a dichroic mirror 530, an alignment light source 520, and a light receiving element 510. In addition, as an example, in the present application example, the alignment optical system 500 may include the lens 540 as an optical element for correcting the magnification of the alignment index.

The alignment light emitted from the alignment light source 520 is reflected by the dichroic mirror 530 and is applied toward the subject eye E through the objective optical system. The alignment light reflected by the cornea of the subject eye E passes through the objective optical system, is reflected by the dichroic mirror 530, and is received by the light receiving element 510 through the lens 540. The light receiving element 510 detects the alignment state of the subject eye E with respect to the objective optical system based on the result of light receiving. The signal output from the light receiving element 510 is input to the control unit 70 (refer to Fig. 4). The control unit 70 may generate an image indicating the alignment state of the subject eye E (hereinafter, also referred to as an "alignment image") based on the input signal. The alignment image may be, for example, an image including an alignment index image indicating the alignment light reflected by the cornea of the subject eye E.

Here, the lens 540 can be moved along the optical axis, and the focal length of the alignment optical system is changed and the magnification of the alignment index image is varied. The fundus imaging device 1 may be provided with a driving unit 540a that moves the lens 540. The lens 540 is displaced according to the attachment and detachment (insertion and removal) of the attachment optical system 330 (that is, according to switching of the angle of view).

As the alignment optical system 500, an anterior segment observation system that images an anterior segment of the subject eye may be used.

Next, an optical configuration when the imaging angle of view is a second angle of view which is a wider angle of view, will be described with reference to Fig. 3. In the present embodiment, the attachment optical system 330 is arranged between the objective optical system 300 and the subject eye E, and thus, the objective optical system 18 is configured for performing the imaging with the second angle of view. The attachment optical system 330 in the present embodiment includes at least one lens. As illustrated in Fig. 3, the attachment optical system 330 may include a plurality of lenses.

When the attachment optical system 330 is attached, since the imaging angle of view is increased, while the magnification is reduced, if the positional relationship between the lens 540 and the light receiving element 510 is maintained before and after attachment and detachment (insertion and removal) of the attachment optical system 330, the alignment index image captured by the light receiving element 510 is reduced. In the present application example, the lens 540 is displaced, and a distance between the focal point of the corneal reflected light of the index light flux and the imaging surface of the light receiving element 510 can be switched. Specifically, if the attachment optical system 330 is not attached (that is, it is the first angle of view), the control unit 70 sets the distance between the focal point of the cornea reflected light of the index light flux and the imaging surface of the light receiving element 510 to a first distance. On the other hand, if the attachment optical system 330 is attached (that is, in the case of the second angle of view), the control unit 70 sets the distance between the focal point of the cornea reflected light of the index light flux and the imaging surface of the light receiving element 510 to a second distance that is longer than the first distance. In this way, the change in the size of the alignment index image based on the switching of the angle of view of the imaging optical system is optically corrected.

The control unit 70 may change the unit displacement amount (adjustment pitch) of the lens 214 (optical element in the diopter correction unit) before and after the attachment and detachment (insertion and removal) of the attachment optical system 330. More specifically, the adjustment pitch may be increased in the insertion state compared to that in the retraction state (not attached). As a result, it becomes easy to shorten the time required for diopter correction.

In addition, the control unit 70 may change the position of the lens 214 before and after the attachment and detachment (insertion and removal) of the attachment optical system 330. In this way, the change in the diopter of the optical system due to the attachment and detachment (insertion and removal) of the attachment optical system 330 may be corrected.

In addition, the control unit 70 may change the detection region where the state of the diopter correction of the SLO optical system 200 is detected before and after the attachment and detachment (insertion and removal) of the attachment optical system 330, which is the detection region on the observation image. For example, in the retraction state, the state of the diopter correction is detected based on the contrast of the entire observation image, and in the insertion state, the state of the diopter correction is detected based on the contrast of the center part of the observation image (here, the region corresponding to an angle of view of 60° similar to that of the observation image in the retraction state). The diopter may be adjusted so that the contrast of the detection region is maximized in each state. In the present application example, in both the insertion state and the retraction state, since the state of the diopter correction for the angle of view of 60° is the same, it is advantageous in associating the fundus images captured between the insertion state and the retraction state or in comparing.

### <Imaging Operation>

Next, the imaging operation by the fundus imaging device 1 will be described. First, the examiner causes the fundus imaging device 1 to attach and detach the attachment optical system 330, and sets the imaging angle of view of the fundus imaging device 1. The attachment and detachment (insertion and removal) state of the attachment optical system 330 may be manually input to the fundus imaging device 1 by the examiner. In addition, a sensor for detecting the attachment and detachment (insertion and removal) state of the attachment optical system 330 may be provided in the fundus imaging device 1, and the detection signal from the sensor may be input to the fundus imaging device 1.

According to the attachment and detachment state of the attachment optical system 330, various switching regarding the OCT optical system 100 such as the amount of light, the gain, the diopter correction amount, the amount of light balance between the measurement light and the reference light, the resolution in the depth direction, and the scanning density between the peripheral part and the center part, may be performed. Furthermore, regarding the SLO optical system 200, various switching such as the amount of light, the gain, the diopter correction amount, the opening of the aperture, and the like, may be performed.

### <Imaging Method Selection Screen>

In addition, the control unit 70 may display a widget for selecting the imaging method on the monitor 75 according to the attachment and detachment (insertion and removal) state of the attachment optical system 330 (refer to Fig. 8). For example, as the imaging method in the retraction state, color imaging, FA imaging, ICGA imaging, spontaneous fluorescence imaging, OCT imaging may be selectable. In the retraction state, a widget (buttons in Fig. 8) for selecting those imaging methods may be displayed on the monitor 75. On the other hand, in the insertion state, as the imaging method, the spontaneous fluorescence imaging may not be selectable, and the color imaging, the FA imaging, the ICGA imaging, and the OCT imaging may be selectable. In the insertion state, the widget for selecting the spontaneous fluorescence imaging may be hidden, and only the widgets for selecting the remaining imaging methods may be displayed on the monitor 75. In this way, in the present application example, the spontaneous fluorescence imaging in the insertion state, which is difficult to image with an appropriate signal efficiency, is hidden in the widget and not selectable, and therefore, it is possible to suppress an image of low clinical significance from being captured.

After the imaging method is selected, the control unit 70 may start to acquire and display the observation image. The examiner confirms the imaging position and the like on the observation image and performs the release operation. As a result, the fundus image is captured by the previously selected imaging method.

As described above, the description is made based on the embodiment, but the present disclosure is not limited to the above embodiment.

### Reference Signs List

- 1: fundus imaging device
- 70: control unit
- 100: OCT optical system
- 102: OCT light source
- 134: scanning unit
- 200: SLO optical system
- 211: laser light source
- 216: scanning unit
- 320: objective lens
- 330: lens attachment

## Claims

1. A fundus imaging device (1) comprising:
an imaging optical system (200) configured to scan light from a light source (210) using a scanning unit (216) and to irradiate a fundus (Er) of a subject eye (E) with the light through an objective optical system (300) to image the fundus (Er) of the subject eye (E)based on return light from the subject eye (E);
angle-of-view switching means (330) configured to switch an angle of view in the imaging optical system (200) between a first angle of view and a second angle of view larger than the first angle of view by changing the objective optical system (300); and
control means (70) configured to change between a first mode for imaging the fundus (Er) at the first angle of view and a second mode for imaging the fundus (Er) at the second angle of view,
wherein the control means (70) is configured to change an imaging condition in the imaging optical system (200) between the first mode and the second mode,
wherein the imaging optical system (200) includes a diopter correction unit (240) configured to drive an optical element (214) to perform a diopter correction according to the subject eye (E), and
the control means (70) is configured to switch control performed by the diopter correction unit (240) between the first mode and the second mode,
**characterised in that** the control means (70) is configured to selectively control the diopter correction unit (240) such that selectively either different layers of the fundus (Er) or the same layers of the fundus (ER) between the first mode and the second mode are focused as a target part.

2. The fundus imaging device (1) according to claim 1,
wherein the control means (70) is configured to set an amount of driving of the optical element per a diopter correction step in each of the first mode and the second mode in order that the diopter correction step (unit: D) by the diopter correction unit (240) is increased in the second mode compared to the first mode.

3. The fundus imaging device (1) according to claim 1 or 2,
wherein the control means (70) is configured to decrease an amount of driving of the optical element (214) per a diopter correction step (unit: D) by the diopter correction unit in the second mode compared to the first mode.

4. The fundus imaging device (1) according to any one of claims 1 to 3,
wherein the imaging optical system (200) is for capturing a front image of the fundus (Er), and
the control means (70) is configured to:
detect a state of the diopter correction based on an observation image of the fundus acquired through the imaging optical system (200);
set a detection region to detect the state of the diopter correction in the observation image;
detect the state of the diopter correction based on the detection region; and
change a range occupied by the detection region on the observation image such that the range differs between the first mode and the second mode.

5. The fundus imaging device (1) according to claim 1,
wherein the imaging optical system (200) is for capturing a front image of the fundus (Er),
the control means (70) is configured to:
set a detection region on an observation image of the fundus acquired through the imaging optical system (200); and
detect a state of the diopter correction based on the detection region, wherein
the detection region in the second mode matches the detection region in the first mode on the fundus.

6. The fundus imaging device (1) according to claim 1,
wherein the imaging optical system (200) includes an OCT optical system (100) that is configured to acquire OCT data of the fundus (Er) based on a spectral interference signal of the return light and reference light, and
the control means (70) is configured to, in the first mode, set a diopter correction amount by the diopter correction unit (240) constant regardless of a scanning position of the light on the fundus (Er), and, in the second mode, changes a diopter correction amount between when the scanning position of the light on the fundus (Er) is at least in a fundus central part and when the scanning position is in a fundus peripheral part.

7. The fundus imaging device (1) according to claim 1,
wherein the control means (70) is configured to control the diopter correction unit (240) to set different reference correction amounts between the first mode and the second mode, and to further set a final correction amount from ranges before and after the reference correction value.

8. The fundus imaging device (1) according to any one of claims 1 to 7,
wherein the control means (70) is configured to change at least one of an amount of the light applied onto and received from the subject eye (E) and a gain of the return light with respect to a received light signal, between the first mode and the second mode.

9. The fundus imaging device (1) according to any one of claims 1 to 8, further comprising:
a fixation optical system (200) is configured to present a fixation target for the subject eye (E), and
wherein the control means (70) is configured to select a presentation position of the fixation target from a plurality of predetermined presentation positions according to a selection operation from the examiner, and to switch the presentation position selectable based on the selection operation, between the first mode and the second mode.

10. The fundus imaging device (1) according to any one of claims 1 to 5, or according to claim 7,
wherein the imaging optical system (200) includes an OCT optical system (100) configured to acquire OCT data of the fundus (Er) based on a spectral interference signal of the return light and reference light, and
the control means (70) is configured to control the OCT optical system (100) to change a resolution in a depth direction between the first mode and the second mode.

11. The fundus imaging device (1) according to claim 10,
wherein a detector (120), which is configured to detect the spectral interference signal in the OCT optical system (100), includes a grading element (121) and a light receiving element (122), and
the control means (70) is configured to change an interval between the grading element (121) and the light receiving element (122), between the first mode and the second mode.

12. The fundus imaging device (1) according to any one of claims 1 to 11, further comprising:
light amount detection means configured to detect an amount of the light applied from the imaging optical system (200) to the subject eye (E),
wherein the control means (70) is configured to:
compare the amount of the light detected by the light amount detection means with a threshold value;
allow the irradiation of the subject eye (E) with the light, in a case where the amount of the light is equal to or smaller than the threshold value;
inhibit the irradiation of the subject eye (E) with the light, in a case where the amount of the light is larger than the threshold value; and
change the threshold value between the first mode and the second mode.

## Patentansprüche

1. Fundusabbildungsvorrichtung (1), umfassend:
ein optisches Abbildungssystem (200), das konfiguriert ist, um Licht von einer Lichtquelle (210) unter Verwendung einer Abtasteinheit (216) abzutasten und einen Fundus (Er) eines Subjektauges (E) mit dem Licht durch ein optisches Objektivsystem (300) zu bestrahlen, um den Fundus (Er) des Subjektauges (E) basierend auf Rückkehrlicht von dem Subjektauge (E) abzubilden;
ein Blickwinkelumschaltmittel (330), das konfiguriert ist, um einen Blickwinkel in dem optischen Abbildungssystem (200) zwischen einem ersten Blickwinkel und einem zweiten Blickwinkel, der größer als der erste Blickwinkel ist, durch Ändern des optischen Objektivsystems (300) umzuschalten; und
ein Steuermittel (70), das konfiguriert ist, um zwischen einem ersten Modus zum Abbilden des Fundus (Er) bei dem ersten Blickwinkel und einem zweiten Modus zum Abbilden des Fundus (Er) bei dem zweiten Blickwinkel zu wechseln,
wobei das Steuermittel (70) konfiguriert ist, um eine Abbildungsbedingung in dem optischen Abbildungssystem (200) zwischen dem ersten Modus und dem zweiten Modus zu ändern,
wobei das optische Abbildungssystem (200) eine Dioptrienkorrektureinheit (240) beinhaltet, die konfiguriert ist, um ein optisches Element (214) anzutreiben, um eine Dioptrienkorrektur gemäß dem Subjektauge (E) durchzuführen, und
das Steuermittel (70) konfiguriert ist, um eine Steuerung, die durch die Dioptrienkorrektureinheit (240) durchgeführt wird, zwischen dem ersten Modus und dem zweiten Modus umzuschalten,
**dadurch gekennzeichnet, dass** das Steuermittel (70) konfiguriert ist, um die Dioptrienkorrektureinheit (240) selektiv zu steuern, sodass selektiv entweder unterschiedliche Schichten des Fundus (Er) oder die gleichen Schichten des Fundus (ER) zwischen dem ersten Modus und dem zweiten Modus als ein Zielteil fokussiert werden.

2. Fundusabbildungsvorrichtung (1) nach Anspruch 1,
wobei das Steuermittel (70) konfiguriert ist, um einen Betrag des Antreibens des optischen Elements pro Dioptrienkorrekturschritt in jedem von dem ersten Modus und dem zweiten Modus einzustellen, damit der Dioptrienkorrekturschritt (Einheit: D) durch die Dioptrienkorrektureinheit (240) in dem zweiten Modus im Vergleich zu dem ersten Modus erhöht wird.

3. Fundusabbildungsvorrichtung (1) nach Anspruch 1 oder 2,
wobei das Steuermittel (70) konfiguriert ist, um einen Betrag des Antreibens des optischen Elements (214) pro Dioptrienkorrekturschritt (Einheit: D) durch die Dioptrienkorrektureinheit in dem zweiten Modus im Vergleich zu dem ersten Modus zu verringern.

4. Fundusabbildungsvorrichtung (1) nach einem der Ansprüche 1 bis 3,
wobei das optische Abbildungssystem (200) zum Aufnehmen eines Vorderbilds des Fundus (Er) dient, und
das Steuermittel (70) konfiguriert ist, um:
einen Zustand der Dioptrienkorrektur basierend auf einem Beobachtungsbild des Fundus zu erfassen, das durch das optische Abbildungssystem (200) erfasst wird;
einen Erfassungsbereich einzustellen, um den Zustand der Dioptrienkorrektur in dem Beobachtungsbild zu erfassen;
den Zustand der Dioptrienkorrektur basierend auf dem Erfassungsbereich zu erfassen, und
einen Bereich zu ändern, der von dem Erfassungsbereich auf dem Beobachtungsbild eingenommen wird, so dass sich der Bereich zwischen dem ersten Modus und dem zweiten Modus unterscheidet.

5. Fundusabbildungsvorrichtung (1) nach Anspruch 1,
wobei das optische Abbildungssystem (200) zum Aufnehmen eines Vorderbilds des Fundus (Er) dient,
das Steuermittel (70) konfiguriert ist, um:
einen Erfassungsbereich auf einem Beobachtungsbild des Fundus einzustellen, das durch das optische Abbildungssystem (200) erfasst wird; und
einen Zustand der Dioptrienkorrektur basierend auf dem Erfassungsbereich zu erfassen, wobei
der Erfassungsbereich in dem zweiten Modus mit dem Erfassungsbereich in dem ersten Modus auf dem Fundus übereinstimmt.

6. Fundusabbildungsvorrichtung (1) nach Anspruch 1,
wobei das optische Abbildungssystem (200) ein optisches OCT-System (100) beinhaltet, das konfiguriert ist, um OCT-Daten des Fundus (Er) basierend auf einem spektralen Interferenzsignal des Rückkehrlichts und Referenzlichts zu erfassen, und
das Steuermittel (70) konfiguriert ist, um in dem ersten Modus einen Dioptrienkorrekturbetrag durch die Dioptrienkorrektureinheit (240) unabhängig von einer Abtastposition des Lichts auf dem Fundus (Er) konstant einzustellen, und in dem zweiten Modus einen Dioptrienkorrekturbetrag zwischen dem Zeitpunkt, wenn sich die Abtastposition des Lichts auf dem Fundus (Er) zumindest in einem Fundusmittelteil befindet, und dem Zeitpunkt, wenn sich die Abtastposition in einem Fundusumfangsteil befindet, wechselt.

7. Fundusabbildungsvorrichtung (1) nach Anspruch 1,
wobei das Steuermittel (70) konfiguriert ist, um die Dioptrienkorrektureinheit (240) zu steuern, um unterschiedliche Referenzkorrekturbeträge zwischen dem ersten Modus und dem zweiten Modus einzustellen, und um ferner einen endgültigen Korrekturbetrag aus Bereichen vor und nach dem Referenzkorrekturwert einzustellen.

8. Fundusabbildungsvorrichtung (1) nach einem der Ansprüche 1 bis 7,
wobei das Steuermittel (70) konfiguriert ist, um mindestens eines von einer Menge des Lichts, das auf das Subjektauge (E) angewendet und von diesem empfangen wird, und einer Verstärkung des Rückkehrlichts in Bezug auf ein empfangenes Lichtsignal zwischen dem ersten Modus und dem zweiten Modus zu ändern.

9. Fundusabbildungsvorrichtung (1) nach einem der Ansprüche 1 bis 8, ferner umfassend:
ein optisches Fixierungssystem (200), das konfiguriert ist, um ein Fixierungsziel für das Subjektauge (E) darzustellen, und
wobei das Steuermittel (70) konfiguriert ist, um eine Darstellungsposition des Fixierungsziels aus einer Vielzahl von vorbestimmten Darstellungspositionen gemäß einem Auswahlvorgang von dem Prüfer auszuwählen, und um die Darstellungsposition, die basierend auf dem Auswahlvorgang auswählbar ist, zwischen dem ersten Modus und dem zweiten Modus umzuschalten.

10. Fundusabbildungsvorrichtung (1) nach einem der Ansprüche 1 bis 5 oder nach Anspruch 7,
wobei das optische Abbildungssystem (200) ein optisches OCT-System (100) beinhaltet, das konfiguriert ist, um OCT-Daten des Fundus (Er) basierend auf einem spektralen Interferenzsignal des Rückkehrlichts und Referenzlichts zu erfassen, und
das Steuermittel (70) konfiguriert ist, um das optische OCT-System (100) zu steuern, um eine Auflösung in einer Tiefenrichtung zwischen dem ersten Modus und dem zweiten Modus zu ändern.

11. Fundusabbildungsvorrichtung (1) nach Anspruch 10,
wobei ein Detektor (120), der konfiguriert ist, um das spektrale Interferenzsignal in dem optischen OCT-System (100) zu erfassen, ein Abstufungselement (121) und ein Lichtempfangselement (122) beinhaltet, und
das Steuermittel (70) konfiguriert ist, um ein Intervall zwischen dem Abstufungselement (121) und dem Lichtempfangselement (122) zwischen dem ersten Modus und dem zweiten Modus zu ändern.

12. Fundusabbildungsvorrichtung (1) nach einem der Ansprüche 1 bis 11, ferner umfassend:
ein Lichtmengenerfassungsmittel, das konfiguriert ist, um eine Menge des Lichts zu erfassen, das von dem optischen Abbildungssystem (200) auf das Subjektauge (E) angewendet wird,
wobei das Steuermittel (70) konfiguriert ist, um:
die Menge des Lichts, die durch das Lichtmengenerfassungsmittel erfasst wird, mit einem Schwellenwert zu vergleichen;
die Bestrahlung des Subjektauges (E) mit dem Licht zu erlauben, in einem Fall, in dem die Menge des Lichts gleich oder kleiner als der Schwellenwert ist;
die Bestrahlung des Subjektauges (E) mit dem Licht zu verhindern, in einem Fall, in dem die Menge des Lichts größer als der Schwellenwert ist; und
den Schwellenwert zwischen dem ersten Modus und dem zweiten Modus zu ändern.

## Revendications

1. Dispositif d'imagerie de fond d'oeil (1) comprenant :
un système optique d'imagerie (200) configuré pour balayer la lumière provenant d'une source de lumière (210) en utilisant une unité de balayage (216) et pour irradier un fond d'oeil (Er) d'un oeil sujet (E) avec la lumière à travers un système optique d'objectif (300) pour imager le fond d'oeil (Er) de l'oeil sujet (E) sur la base de la lumière de retour provenant de l'oeil sujet (E) ;
un moyen de commutation d'angle de vision (330) configuré pour commuter un angle de vision dans le système optique d'imagerie (200) entre un premier angle de vision et un second angle de vision plus grand que le premier angle de vision en changeant le système optique d'objectif (300) ; et
un moyen de commande (70) configuré pour changer entre un premier mode pour imager le fond d'oeil (Er) au premier angle de vision et un second mode pour imager le fond d'oeil (Er) au second angle de vision,
dans lequel le moyen de commande (70) est configuré pour changer une condition d'imagerie dans le système optique d'imagerie (200) entre le premier mode et le second mode,
dans lequel le système optique d'imagerie (200) comprend une unité de correction de dioptrie (240) configurée pour entraîner un élément optique (214) pour effectuer une correction de dioptrie selon l'oeil sujet (E), et
le moyen de commande (70) est configuré pour commuter la commande effectuée par l'unité de correction de dioptrie (240) entre le premier mode et le second mode,
**caractérisé en ce que** le moyen de commande (70) est configuré pour commander sélectivement l'unité de correction de dioptrie (240) de sorte que sélectivement soit différentes couches du fond d'oeil (Er) soit les mêmes couches du fond d'oeil (ER) entre le premier mode et le second mode soient focalisées en tant que partie cible.

2. Dispositif d'imagerie de fond d'oeil (1) selon la revendication 1,
dans lequel le moyen de commande (70) est configuré pour régler une quantité d'entraînement de l'élément optique par étape de correction de dioptrie dans chacun du premier mode et du second mode afin que l'étape de correction de dioptrie (unité : D) par l'unité de correction de dioptrie (240) soit augmentée dans le second mode par rapport au premier mode.

3. Dispositif d'imagerie de fond d'oeil (1) selon la revendication 1 ou 2,
dans lequel le moyen de commande (70) est configuré pour diminuer une quantité d'entraînement de l'élément optique (214) par étape de correction de dioptrie (unité : D) par l'unité de correction de dioptrie dans le second mode par rapport au premier mode.

4. Dispositif d'imagerie de fond d'oeil (1) selon l'une quelconque des revendications 1 à 3,
dans lequel le système optique d'imagerie (200) est destiné à capturer une image frontale du fond d'oeil (Er), et
le moyen de commande (70) est configuré pour :
détecter un état de la correction de dioptrie sur la base d'une image d'observation du fond d'oeil acquise par l'intermédiaire du système optique d'imagerie (200) ;
régler une région de détection pour détecter l'état de la correction de dioptrie dans l'image d'observation ;
détecter l'état de la correction de dioptrie sur la base de la région de détection ; et
changer une plage occupée par la région de détection sur l'image d'observation de sorte que la plage diffère entre le premier mode et le second mode.

5. Dispositif d'imagerie de fond d'oeil (1) selon la revendication 1,
dans lequel le système optique d'imagerie (200) est destiné à capturer une image frontale du fond d'oeil (Er),
le moyen de commande (70) est configuré pour :
régler une région de détection sur une image d'observation du fond d'oeil acquise par l'intermédiaire du système optique d'imagerie (200) ; et
détecter un état de la correction de dioptrie sur la base de la région de détection, dans lequel
la région de détection dans le second mode correspond à la région de détection dans le premier mode sur le fond d'oeil.

6. Dispositif d'imagerie de fond d'oeil (1) selon la revendication 1,
dans lequel le système optique d'imagerie (200) comprend un système optique OCT (100) qui est configuré pour acquérir des données OCT du fond d'oeil (Er) sur la base d'un signal d'interférence spectrale de la lumière de retour et de la lumière de référence, et
le moyen de commande (70) est configuré pour, dans le premier mode, régler une quantité de correction de dioptrie par l'unité de correction de dioptrie (240) constante indépendamment d'une position de balayage de la lumière sur le fond d'oeil (Er), et, dans le second mode, change une quantité de correction de dioptrie entre le moment où la position de balayage de la lumière sur le fond d'oeil (Er) est au moins dans une partie centrale de fond d'oeil et le moment où la position de balayage est dans une partie périphérique de fond d'oeil.

7. Dispositif d'imagerie de fond d'oeil (1) selon la revendication 1,
dans lequel le moyen de commande (70) est configuré pour commander l'unité de correction de dioptrie (240) pour régler différentes quantités de correction de référence entre le premier mode et le second mode, et pour régler en outre une quantité de correction finale à partir de plages avant et après la valeur de correction de référence.

8. Dispositif d'imagerie de fond d'oeil (1) selon l'une quelconque des revendications 1 à 7,
dans lequel le moyen de commande (70) est configuré pour changer au moins l'un d'une quantité de la lumière appliquée sur et reçue de l'oeil sujet (E) et d'un gain de la lumière de retour par rapport à un signal lumineux reçu, entre le premier mode et le second mode.

9. Dispositif d'imagerie de fond d'oeil (1) selon l'une quelconque des revendications 1 à 8, comprenant en outre :
un système optique de fixation (200) est configuré pour présenter une cible de fixation pour l'oeil sujet (E), et
dans lequel le moyen de commande (70) est configuré pour sélectionner une position de présentation de la cible de fixation parmi une pluralité de positions de présentation prédéterminées selon une opération de sélection de l'examinateur, et pour commuter la position de présentation sélectionnable sur la base de l'opération de sélection, entre le premier mode et le second mode.

10. Dispositif d'imagerie de fond d'oeil (1) selon l'une quelconque des revendications 1 à 5, ou selon la revendication 7,
dans lequel le système optique d'imagerie (200) comprend un système optique OCT (100) configuré pour acquérir des données OCT du fond d'oeil (Er) sur la base d'un signal d'interférence spectrale de la lumière de retour et de la lumière de référence, et
le moyen de commande (70) est configuré pour commander le système optique OCT (100) pour changer une résolution dans une direction de profondeur entre le premier mode et le second mode.

11. Dispositif d'imagerie de fond d'oeil (1) selon la revendication 10,
dans lequel un détecteur (120), qui est configuré pour détecter le signal d'interférence spectrale dans le système optique OCT (100), comprend un élément de gradation (121) et un élément de réception de lumière (122), et
le moyen de commande (70) est configuré pour changer un intervalle entre l'élément de gradation (121) et l'élément de réception de lumière (122), entre le premier mode et le second mode.

12. Dispositif d'imagerie de fond d'oeil (1) selon l'une quelconque des revendications 1 à 11, comprenant en outre :
un moyen de détection de quantité de lumière configuré pour détecter une quantité de la lumière appliquée du système optique d'imagerie (200) à l'oeil sujet (E),
dans lequel le moyen de commande (70) est configuré pour :
comparer la quantité de la lumière détectée par le moyen de détection de quantité de lumière à une valeur de seuil ;
permettre l'irradiation de l'oeil sujet (E) avec la lumière, dans un cas où la quantité de la lumière est inférieure ou égale à la valeur de seuil ;
empêcher l'irradiation de l'oeil sujet (E) avec la lumière, dans un cas où la quantité de la lumière est supérieure à la valeur de seuil ; et
changer la valeur de seuil entre le premier mode et le second mode.
